# EUROPEAN PATENT APPLICATION

(11) **EP 3 967 278 A1**
(43) Date of publication of application: **16.03.2022**
(21) Application number: 20806335.4
(22) Date of filing: 02.04.2020
(51) Int. Cl.: A61F 2/90

(54) **BLOOD VESSEL STENT**

(30) Priority: 10.05.2019 CN 201910390356
(71) Applicant: Shanghai Bluevascular Medtech Co., Ltd., Shanghai 200120 (CN)
(72) Inventor: WANG, Liwen, Shanghai 200120 (CN); HUANG, Dingguo, Shanghai 200120 (CN); ZHU, Qing, Shanghai 200120 (CN); ZHANG, Linlin, Shanghai 200120 (CN); ZHANG, Zhaoduo, Shanghai 200120 (CN)
(74) Representative: Arnold & Siedsma
(86) International application number: PCT/CN2020/082923
(87) International publication number: WO 2020/228440

(57) **Abstract**

Disclosed is a blood vessel stent, comprising a stent body. The stent body is a reticulated tube structure woven by metal wires, wherein the stent body comprises a first stent segment and a second stent segment that are connected in the axial direction, and the mesh density of the first stent segment is different from that of the second stent segment. The blood vessel stent provided in the present invention can improve the treatment effect for involving inferior vena cava and iliofemoral vein stenosis by means of bare sections, can improve the support strength and anti-compression performance for a compression part by means of a dense mesh structure having a larger mesh density, can increase the flexibility and wall attachment performance for a bending part by means of a sparse mesh structure having a smaller mesh density, can adapt to the shapes of blood vessels for different anatomical shapes of blood vessels by means of a diameter variable structure, and is suitable for various blood vessel diameters.

## Description

### TECHNICAL FIELD

The present application relates to the field of vascular intervention medicine, and particularly to a vascular stent.

### BACKGROUND

Venous disease is currently a common disease in vascular surgery diseases, especially iliac vein compression syndrome, which refers to a syndrome in which iliac artery compression leads to intracavitary adhesion, stenosis, or occlusion, resulting in various clinical symptoms. The incidence of iliac vein compression syndrome is 20% to 40%.

In recent years, interventional therapy has been used as a routine procedure for the treatment of venous compression. At present, there are cutting stents and woven stents for the treatment of venous stenosis. The cutting stent can support the stenosis and occlusion section of the blood vessel, reduce the elastic retraction, and keep the blood flow in the lumen smooth. However, there are some shortcomings, such as insufficient flexibility and easy breakage. The woven stent is a mesh tubular structure woven and wound by a wire or multiple wires. The woven stent has received more and more attention due to its good flexibility, strong support strength and good adhesion.

### SUMMARY

### Technical problem

The technical problem to be solved by the present application is to provide a vascular stent which has strong support strength and excellent flexibility.

### Technical solutions

The technical solutions adopted by the present application to solve the above technical problem is to provide a vascular stent including a stent body, the stent body is a mesh tubular structure woven by metal wires; the stent body includes a first stent section and a second stent section connected in an axial direction; a mesh density of the first stent section is different from a mesh density of the second stent section.

Further, the stent body has a variable-diameter structure in the axial direction, and a proximal diameter D1 of the stent body is greater than a distal diameter D2.

Further, the proximal diameter D1 of the stent body is 8 to 22 mm, and the distal diameter D2 of the stent body is 6 to 22 mm.

Further, the mesh of the stent body is a diamond-shaped mesh; the side length A of the diamond-shaped mesh is 1 to 5 mm, and the angle β of the diamond-shaped mesh is 90° to 160° .

Further, a shorter one of two diagonals of the diamond-shaped mesh in the first stent section has a length of 1 to 4 mm, and a shorter one of two diagonals of the diamond-shaped mesh in the second stent section has a length of 2 to 6 mm

Further, the vascular stent further includes a first bare section, the first bare section includes at least one first protrusion woven by metal wires in a circumferential direction; a distal end of the first bare section is connected to a proximal end of the first stent section, and a distal end of the first stent section is connected to a proximal end of the second stent section

Further, the proximal end of the first stent section includes at least one third protrusion woven by metal wires in the circumferential direction; a vertex of the first protrusion of the first bare section and a vertex of the third protrusion at the proximal end of the first stent section is on the same axis.

Further, the vertex of the first protrusion at the proximal end of the first bare section and the vertex of the third protrusion at the proximal end of the first stent section are evenly spaced along the circumferential direction of the vascular stent.

Further, a length L3 of the first bare section in the axial direction is 1 to 10 mm

Further, a number of the first protrusions of the first bare section is 1 to 10.

Further, the vascular stent further includes a second bare section, the second bare section includes at least one second protrusion woven by metal wires in the circumferential direction; a proximal end of the second bare section is connected to a distal end of the second stent section.

Further, the first bare section and the first stent section partially overlap in the axial direction, and the first bare section partially extends beyond the first stent section in the axial direction.

Further, an angle between an extension direction of the first bare section and an axial direction of the vascular stent is 0° to 60°.

Further, the mesh density of the first bare section and the second stent section are both less than the mesh density of the first stent section.

### Beneficial effect

Compared with the prior art, the present application has the following beneficial effects: in the vascular stent according to the present application, the vascular stent body includes the first stent section and the second stent section with different mesh densities, and the change in mesh density is beneficial for use in venous diseased areas at different locations. For example, for the iliac vein, the right iliac vein is compressed by the right iliac artery. This position is located at the beginning of the right iliac vein, which requires high extrusion resistance. Therefore, the stent structure here can be a high-density mesh woven structure, and the area can be woven with high-density in the later stage according to the anatomical structure that requires strong support strength and extrusion resistance. According to different needs, the degree of mesh density can be different. A woven structure with low mesh density can be used for areas with high bending performance. Larger meshes can increase bending performance and flexibility to better fit the shape of blood vessels. In addition, the stent body is configured as a variable-diameter stent, which can better conform to the shape of blood vessels. In particular, the first bare section provided at the proximal end of the stent body allows the overall stent to extend from the iliac vein into the inferior vena cava without affecting the blood flow of the contralateral vein, and ensuring the integrity of the stent after being placed in the vein. The second bare section provided at the distal end can cross other vein branches to avoid affecting the blood flow of other branches. The vascular stent of vein provided by the present application can treat various venous diseases. For stenosis involving inferior vena cava and iliac vein, there is a bare section that can improve the treatment effect. For the compressed part, there is a dense mesh tubular structure that can improve the support strength and extrusion resistance. For the bending part, there is a sparse mesh tubular structure with large meshes that can increase the flexibility and adhesion. For the anatomical shape of the blood vessel, there is a variable-diameter structure that can conform to the shape of the blood vessel, which is suitable for various blood vessel diameters.

### BRIEF DESCRIPTION OF THE DRAWINGS

Fig. 1 is a schematic diagram of a vascular stent according to an embodiment of the present application;
Fig. 2 is a schematic diagram of mesh cells according to an embodiment of the present application;
Fig. 3 is a schematic diagram of a compressed vein according to an embodiment of the present application;
Fig. 4 is a schematic diagram of the use state of the vascular stent implanted in the human body according to an embodiment of the present application;
Fig. 5(a) to Fig. 5(d) are schematic diagrams of partial distribution of a first bare section according to an embodiment of the present application.

In the figures:
1 vascular stent; 2 vein; 3 artery; 10 stent body; 11 first stent section; 12 second stent section; 13 first bare section; 131 first protrusion; 14 mesh cell.

### DETAILED DESCRIPTION

The present application will be further described below with reference to the drawings and embodiments.

In order to describe the structural features of the present application more clearly, "proximal" and "distal" are used as orientation terms, where "proximal" represents an end close to the heart, and "distal" represents an end far away the heart. The term "or" is usually used in the meaning including "and/or" unless specified otherwise clearly in the content.

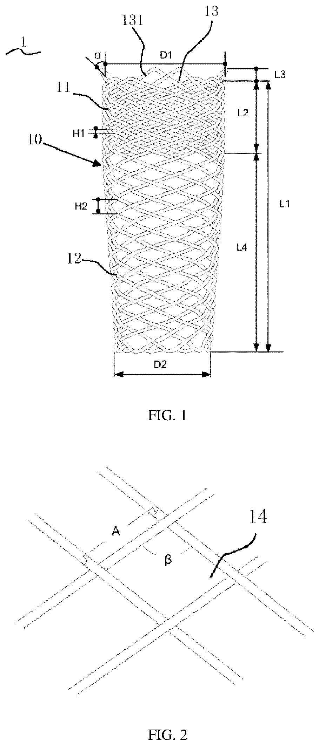

Referring to Fig. 1 and Fig. 2, a vascular stent 1 provided in the present embodiment includes a stent body 10. The stent body 10 is wound into a mesh tubular structure by metal wires, and has a plurality of mesh cells 14. The stent body 10 includes a first stent section 11 and a second stent section 12 connected in an axial direction. The proximal area of the stent body 10 is woven into the first stent section 11 by metal wires. The first stent section 11 has a smaller mesh diameter and a larger mesh density. The distal area of the stent body 10 is woven into the second stent section 12 by metal wires. The second stent section 12 has a larger mesh diameter and a smaller mesh density. The compressed part of the vein requires strong support, and the mesh of the stent body 10 can be densified to promote the support strength and local extrusion resistance; while the uncompressed part and the cross-joint require better flexibility, and the mesh of the stent here can be enlarged to promote the flexibility and adhesion. Specifically, L2 is the first stent section 11 in Fig. 1. The mesh of this area is small, and the distance HI between two opposite vertexes in an axial direction of the mesh cells in this area is preferably 1 to 4 mm. L4 is the second stent section 12. The mesh of the area is large, and the distance H2 between two opposite vertexes in an axial direction of the mesh cells in this area is preferably 2 to 6 mm.

The stent body 10 can be a variable-diameter or equal-diameter structure in an axial direction. The variable-diameter stent is more in line with vein anatomical structure and more suitable for the feature of venous blood vessels. The variable-diameter stent has greater advantages for long-section lesions, and can better comply with the shape of blood vessels. The equal-diameter stent can be applied to a small number of lesions. The equal-diameter stent can be applied to the lesions which are short and has little changes in the diameter. In an embodiment, the stent body 10 is a variable-diameter stent in an axial direction. A proximal diameter D1 of the stent body 10 is preferably 8 to 22 mm, and a distal diameter D2 of the stent body 10 is preferably 6 to 22 mm. In order to comply with the shape of the blood vessel, preferably, the difference between the proximal diameter D1 and the distal diameter D2, that is, the reduced diameter size may be 2 mm or 4 mm

The metal wires for weaving the vascular stent 1 may be memory alloy, stainless steel, titanium alloy, nickel alloy, cobalt-chromium alloy and biodegradable material. Preferably, the stent body 10 is woven by NiTi (nickel titanium) wire, and the wire diameter is between 0.1 and 0.5 mm. The entire vascular stent 1 can be woven by one wire or multiple wires. One metal wire weaving has few connection points; while multiple metal wires weaving has good adhesion and high processing efficiency.

Referring to Fig. 2, preferably, the mesh cells 14 woven by the metal wires is a diamond-shaped mesh. The side length of the diamond-shaped mesh is A, and the angle of the diamond is β. These two parameters and the number of the metal wires used in the stent body 10 affect each other. When the number of metal wires used in the stent body 10 is fixed, the shorter the side length A, the greater the angle β, the stronger the support strength/extrusion resistance, and the better the bending performance for the manufactured stent; the longer the side length A, the smaller the angle β, the weaker the support strength/extrusion resistance, and the poorer bending performance for the stent. When the side length A is fixed, the more the number of wires used in the stent, the smaller the angle β, the weaker the support strength/extrusion resistance, and the poorer bending performance for the stent; on the contrary, the less the number of metal wires, the larger the angle β, the stronger the support strength/extrusion resistance, and the better the bending performance for the stent. When the angle β is fixed, the more the number of metal wires, the shorter the side length A, the stronger the support strength/extrusion resistance, and the better the bending performance for the stent; on the contrary, the less the number of metal wires, the longer the side length A, the weaker the support strength/extrusion resistance, and the poorer the bending performance for the stent. After testing, in addition to strong support strength and extrusion resistance, excellent bending performance and flexibility are also required for venous stents. Therefore, in a preferred embodiment, the number of metal wires is preferably 8-32, the length of the side length A is between 1 and 5 mm, and the angle β is between 90° and 160°. Better implementation results can be achieved by the matching of these three parameters.

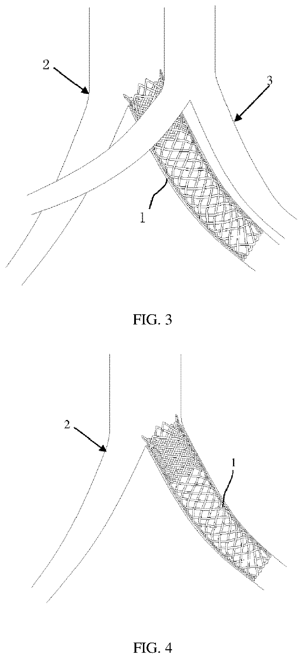

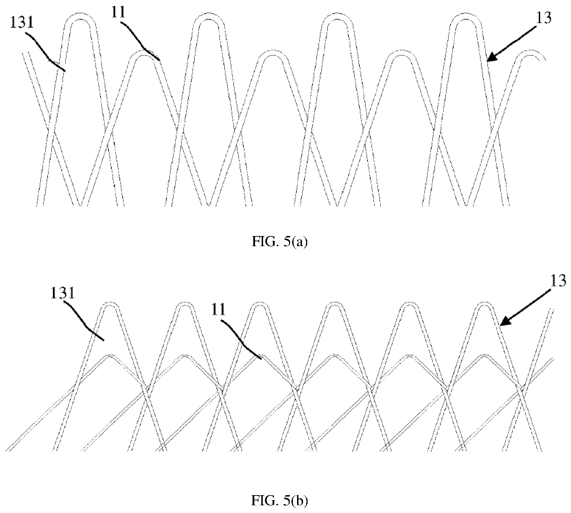

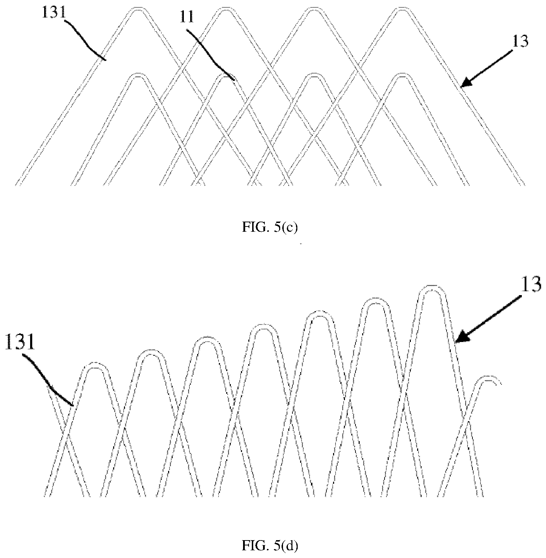

Referring to Fig. 3 and Fig. 4, if an inferior vena cava disease is involved, the vascular stent 1 will be extended to the inferior vena cava 2. If the extension is too long, the blood flow of the contralateral vein will be affected in the long term. If the stent extent is too dense, the blood flow of the contralateral vein will be also affected, and the contralateral vein will be caused to occlude in the long term. Preferably, the proximal end of the stent body 10 is provided with a first bare section 13. The first bare section 13 includes at least one first protrusion 131 woven by metal wires in a circumferential direction. A distal end of the first bare section 13 is connected to a proximal end of the first stent section 11, and a distal end of the first stent section 11 is connected to a proximal end of the second stent section 12. In an embodiment, the first bare section 13 and the first stent section 11 partially overlap in the axial direction, and the first bare section 13 partially extends beyond the first stent section 11 in the axial direction. A distal end of the stent body 10 is provided with a second bare section (not shown). The second bare section includes at least one second protrusion (not shown) woven by metal wires in the circumferential direction. A proximal end of the second bare section is connected to a distal end of the second stent section 12, and the distal end of the first stent section 11 is connected to the proximal end of the second stent section 12. The proximal first bare section 13 can effectively treat iliac-inferior cavity lesions. The length of the first bare section 13 is not long and the number is less, which will not affect the blood flow of the contralateral vein. The distal second bare section can expand venous branches involving distal lesions without affecting the blood flow of other branches. Referring to Fig. 1, the length L3 of the first protrusion 131 in the axial direction is preferably 1 to 10 mm. An angle α between the extending direction of the first protrusion 131 and the axial direction of the stent body can be 0-60°. The number of the first protrusions 131 is controlled within 1 to 10, and the relationship between the first bare section 13 and a proximal vertex of the first stent section 11 is shown in Fig. 5. There are several main structures.

The proximal end of the first stent section includes at least one third protrusion woven by metal wires in the circumferential direction. A vertex of the first protrusion at the proximal end of the first bare section 13 and a vertex of the third protrusion at the proximal end of the first stent section 11 can be on the same axis, and this structure has strong applicability. The vertex of the third protrusion at the proximal end of the first stent section 11 can also be between the vertexes of the first protrusion at the proximal end of the two adjacent first bare sections 13, and this structure has good adhesion. Preferably, the vertex of the first protrusion at the proximal end of the first bare section 13 and the vertex of the third protrusion at the proximal end of the first stent section 11 evenly spaced along the circumferential direction of the vascular stent 1. Since the vein anatomical structure is elliptical, the first bare section 13 can also be designed as a heightened structure, a contoured structure or a spiral structure, etc., to adapt to different vascular shapes. Several structures shown in Figs 5(a) to 5(d) can be extended into the inferior vena cava. The first bare section 13 in Fig. 5(a) is a separate structure. Specifically, the vertex at the proximal end of the first stent section 11 is between the vertexes at the proximal end of the two adjacent first bare sections 13, and the height of each first protrusion 131 in the axial direction is the same. This structure can not only extend the vascular stent 1 into the inferior vena cava to ensure its vascular shape, but also ensure the adhesion of the vascular stent 1 to the iliac vein. In Figs 5(b) and (c), the vertex of the first protrusion at the proximal end of the first bare section 13 and the vertex of the third protrusion at the proximal end of the first stent section 11 are located on the same axis, and the height of the vertexes of each first protrusion 131 in the axial direction is the same. This structure can make the vascular stent 1 have good adhesion performance, strong support strength and extrusion resistance. The first bare section 13 in Fig. 5(d) is a heightened structure. Specifically, the height of the vertex of the first protrusion 131 in the axial direction increases gradually. After the vascular stent 1 of this structure is implanted in the inferior vena cava, the short first protrusion 131 can be located at the bifurcation of the inferior vena cava, and the long first protrusion 131 can be located at the straight section of the inferior vena cava, which facilitates smooth blood flow in the contralateral vein. The first bare section 13 can be woven into different structures according to requirements. In addition to the first bare section 13 at the proximal end, the second bare section can also be added at the distal end to avoid affecting the blood flow of other branches. The structure, number, and shape of the second bare section are similar to those of the first bare section 13, and different structures are woven according to requirements.

The vascular stent 1 provided by the present application has a change in mesh density, which is beneficial for use in venous diseased areas at different locations. For example, for the iliac vein, the right iliac vein is compressed by the stent body of the right iliac. This position is located at the beginning of the right iliac vein, which requires high extrusion resistance. Therefore, the stent structure here can be a high-density mesh woven structure, and the area can be woven with high-density in the later stage according to the anatomical structure that requires strong support strength and extrusion resistance. According to different clinical needs, the degree of change of mesh density can be different. A woven structure with low mesh density can be used for areas with high bending performance. Larger meshes can increase bending performance and flexibility to better fit the shape of blood vessels. In addition, it is configured as a variable-diameter stent, which can better conform to the shape of blood vessels. In particular, the first bare section 13 provided at the proximal end of the stent body 10 allows the overall stent to extend from the iliac vein into the inferior vena cava without affecting the blood flow of the contralateral vein, and ensuring the integrity of the stent after being placed in the vein. The second bare section provided at the distal end can cross other vein branches to avoid affecting the blood flow of other branches. The venous stent provided by the present application can treat various venous diseases. For stenosis involving inferior vena cava and iliac vein, there is a bare section that can improve the treatment effect. For the compressed part, there is a first stent section 11 that can improve the support strength and extrusion resistance. For the bending part, there is a second stent section 12 with large mesh that can increase the flexibility and adhesion. For the anatomical shape of the blood vessel, there is a variable-diameter structure that can conform to the shape of the blood vessel, which is suitable for various blood vessel diameters.

Although the present application has been disclosed as above with the preferred embodiments, it is not intended to limit the present application. Any person skilled in the art can make some modifications and improvements without departing from the spirit and scope of the present application. Therefore, the protection scope of the present application shall be defined in the claims.

## Claims

1. A vascular stent, comprising: a stent body, wherein the stent body is a mesh tubular structure woven by metal wires; **characterized in that** the stent body comprises a first stent section and a second stent section connected in an axial direction; a mesh density of the first stent section is different from a mesh density of the second stent section.

2. The vascular stent according to claim 1, wherein the stent body has a variable-diameter structure in the axial direction, and a proximal diameter D1 of the stent body is greater than a distal diameter D2.

3. The vascular stent according to claim 2, wherein the proximal diameter D1 of the stent body is 8 to 22 mm, and the distal diameter D2 of the stent body is 6 to 22 mm.

4. The vascular stent according to claim 1, wherein the mesh of the stent body is a diamond-shaped mesh; the side length A of the diamond-shaped mesh is 1 to 5 mm, and the angle β of the diamond-shaped mesh is 90° to 160°.

5. The vascular stent according to claim 4, wherein a shorter one of two diagonals of the diamond-shaped mesh in the first stent section has a length of 1 to 4 mm, and a shorter one of two diagonals of the diamond-shaped mesh in the second stent section has a length of 2 to 6 mm

6. The vascular stent according to claim 1, further comprising a first bare section, wherein the first bare section comprises at least one first protrusion woven by metal wires in a circumferential direction; a distal end of the first bare section is connected to a proximal end of the first stent section, and a distal end of the first stent section is connected to a proximal end of the second stent section.

7. The vascular stent according to claim 6, wherein the proximal end of the first stent section comprises at least one third protrusion woven by metal wires in the circumferential direction; a vertex of the first protrusion of the first bare section and a vertex of the third protrusion at the proximal end of the first stent section is on the same axis.

8. The vascular stent according to claim 6, wherein the vertex of the first protrusion at the proximal end of the first bare section and the vertex of the third protrusion at the proximal end of the first stent section are evenly spaced along the circumferential direction of the vascular stent.

9. The vascular stent according to claim 6, wherein a length L3 of the first bare section in the axial direction is 1 to 10 mm

10. The vascular stent according to claim 6, wherein a number of the first protrusions of the first bare section is 1 to 10.

11. The vascular stent according to claim 6, wherein the first bare section and the first stent section partially overlap in the axial direction, and the first bare section partially extends beyond the first stent section in the axial direction.

12. The vascular stent according to claim 6, wherein an angle between an extension direction of the first bare section and an axial direction of the vascular stent is 0° to 60°.

13. The vascular stent according to claim 6, wherein a mesh density of the first bare section and the mesh density of the second stent section are both less than the mesh density of the first stent section.

14. The vascular stent according to any one of claims 1 to 13, further comprising a second bare section; wherein the second bare section comprises at least one second protrusion woven by metal wires in the circumferential direction; a proximal end of the second bare section is connected to a distal end of the second stent section.
